# EUROPEAN PATENT APPLICATION

(11) **EP 3 593 803 A2**
(43) Date of publication of application: **15.01.2020**
(21) Application number: 18764805.0
(22) Date of filing: 12.03.2018
(51) Int. Cl.: A61K 31/625, A61P 13/04

(54) **COMPOUNDS FOR THE TREATMENT OF DISEASES CAUSED BY OXALATE ACCUMULATION**

(30) Priority: 10.03.2017 ES 201730326
(71) Applicant: Universidad de Granada, 18071 Granada (ES); Universidad De La Laguna, 38200 San Cristóbal de La Laguna Tenerife (ES)
(72) Inventor: DÍAZ GAVILÁN, Mónica, 18071 Granada (ES); GÓMEZ VIDAL, José Antonio, 18071 Granada (ES); MOYA GARZÓN, María Dolores, 18071 Granada (ES); SALIDO RUIZ, Eduardo, 38200 San Cristóbal de La Laguna Tenerife (ES); MARTÍN HIGUERAS, Cristina, 38200 San Cristóbal de La Laguna Tenerife (ES); FERNANDES, Miguel Xavier, 38200 San Cristóbal de La Laguna Tenerife (ES)
(74) Representative: Carvajal y Urquijo, Isabel
(86) International application number: PCT/ES2018/070184
(87) International publication number: WO 2018/162785

(57) **Abstract**

The present invention relates to the use of derivatives of salicylic acid for the treatment of diseases or conditions linked to GO and/or PRODH2 enzyme activity, in particular diseases linked to an excess of oxalate, and for the treatment of patients with renal insufficiency (uremia or azotaemia) receiving haemodialysis or peritoneal dialysis, in particular patients treated with ascorbic acid (vitamin C), which is metabolised to oxalate, or patients with fibromyalgia and vulvar pain.

## Description

### SECTOR OF THE ART

The present invention is generally comprised in the field of pharmaceutical chemistry. Specifically, compounds derived from salicylic acid and their application for the treatment of diseases caused by GO and/or PRODH2 enzyme activity, more specifically, diseases caused by excessive oxalate production or accumulation, are described.

### STATE OF THE ART

### Hyperoxalurias

The term hyperoxaluria refers to a high concentration of oxalate in urine. This situation may have a number of different causes. In reference to these causes, hyperoxalurias are classified as primary and secondary.

Primary hyperoxalurias (PH) are a group of autosomal recessive genetic disorders involving enzyme failures that lead to an endogenous surplus production of oxalate. Three types of PH have been described (PH1, PH2 and PH3), with PH1 being the most common and the most aggressive. [(1) Bhasin, B. Primary and Secondary Hyperoxaluria: Understanding the Enigma. World Journal of Nephrology 2015, 4 (2), 235]. The same genetic error that gives rise to PH3 has also been linked to idiopathic oxalate lithiasis. [(1) Monico, C. G.; Rossetti, S.; Belostotsky, R.; Cogal, A. G.; Herges, R. M.; Seide, B. M.; Olson, J. B.; Bergstrahl, E. J.; Williams, H. J.; Haley, W. E.; et al. Primary Hyperoxaluria Type III Gene HOGA1 (Formerly DHDPSL) as a Possible Risk Factor for Idiopathic Calcium Oxalate Urolithiasis. CJASN 2011, 6 (9), 2289-2295.]

Secondary hyperoxalurias may be due to an excessive absorption of oxalate or its precursors in the bowel. This is linked to a diet rich in said precursors, or in the case of enteric hyperoxaluria, to an absorption disorder after bowel resection. [(1) Cochat, P.; Rumsby, G. Primary Hyperoxaluria. New England Journal of Medicine 2013, 369 (7), 649-658. (2) Lorenz, E. C.; Michet, C. J.; Milliner, D. S.; Lieske, J. C. Update on Oxalate Crystal Disease. Curr Rheumatol Rep 2013, 15 (7), 340. (3) Karaolanis, G.; Lionaki, S.; Moris, D.; Palla, V.-V.; Vernadakis, S. Secondary Hyperoxaluria: A Risk Factor for Kidney Stone Formation and Renal Failure in Native Kidneys and Renal Grafts. Transplantation Reviews 2014, 28 (4), 182-187].

### Primary hyperoxaluria type 1

Primary hyperoxaluria type 1 (PH-1) is a serious hereditary disease due to a deficiency of the AGT enzyme (encoded by the Agxt1 gene) in hepatocytes [Zhang, X.; Roe, S.M.; Hou, Y.; Bartlam, M.; Rao, Z.; Pearl, L.H.; Danpure, C.J. J. Mol. Biol. 2003, 331, 643-652].

This enzyme, AGT, is in charge of metabolising glyoxylate in hepatic peroxisomes by transamination into glycine. In PH-1, where AGT activity is absent or the enzyme is erroneously located in the mitochondria, glyoxylate accumulation occurs as a result. Glyoxylate, then, comes to be metabolised by oxidation to oxalate, a process that is mainly catalysed by glycolate oxidase (GO) enzymes in peroxisomes and lactate dehydrogenase (LDH) in the cytoplasm. An excess production of oxalate, which can only be excreted in urine, produces renal capacity saturation and causes oxalate to precipitate in the form of insoluble calcium oxalate crystals. These crystals damage the renal tissue, reducing the excretory capacity of the kidney until ultimately leading to terminal kidney disease. As kidney damage progresses, oxalate accumulation becomes widespread and causes blood vessel, bone, joint, retinal, skin, bone marrow, heart and central nervous system disorders, ultimately leading to the patient's death.

PH-1 is a rare disease with an estimated incidence in Europe of 1:100000 births per year [Cochat, P.; Hulton, S.A.; Acquaviva, C.; Danpure, C. J.; Daudon, M.; Marchi, M. D.; Fargue, S.; Groothoff, J.; Harambat, J.; Hoppe, B.; et al. Nephrol. Dial. Transplant. 2012, 27, 1729-1736.], but it presents at an unusually high frequency in the Canary Islands [(1) Lorenzo, V.; Alvarez, A.; Torres, A.; Torregrosa, V.; Hernández, D.; Salido, E. Kidney Int. 2006, 70, 1115-1119. (2) Santana, A.; Salido, E.; Torres, A.; Shapiro, L. J. PNAS 2003, 100, 7277-7282].

### Treatment of Hyperoxaluria

There is currently no effective pharmacological treatment for hyperoxaluria, and in particular for PH-1. Patients are treated by consuming large amounts of fluids and administering treatments with citrates to increase oxalate solubility in urine. Only in certain cases of PH-1 does the administration of pyridoxine restore AGT activity by redirecting it to its proper location in peroxisomes [(1) Monico, C. G.; Rossetti, S.; Olson, J. B.; Milliner, D. S. Pyridoxine Effect in Type I Primary Hyperoxaluria is Associated with the Most Common Mutant Allele. Kidney Int. 2005, 67 (5), 1704-1709. (2) Fargue, S.; Rumsby, G.; Danpure, C. J. Multiple Mechanisms of Action of Pyridoxine in Primary Hyperoxaluria Type 1. Biochimica et Biophysica Acta (BBA) - Molecular Basis of Disease 2013, 1832 (10), 1776-1783. (3) Salido, E.; Pey, A. L.; Rodriguez, R.; Lorenzo, V. Primary Hyperoxalurias: Disorders of Glyoxylate Detoxification. Biochimica et Biophysica Acta (BBA) - Molecular Basis of Disease 2012, 1822 (9), 1453-1464]. A kidney transplant (palliative) and/or liver transplant (curative) are required as life-saving treatments in PH patients [(1) Zhang, X.; Roe, S.M.; Hou, Y.; Bartlam, M.; Rao, Z.; Pearl, L.H.; Danpure, C.J. J. Mol. Biol. 2003, 331, 643-652. (2) Beck, B. B.; Hoyer-Kuhn, H.; Göbel, H.; Habbig, S.; Hoppe, B. Hyperoxaluria and Systemic Oxalosis: An Update on Current Therapy and Future Directions. Expert Opinion on Investigational Drugs 2013, 22 (1), 117-129. (3) Watts, R. W. E.; Danpure, C. J.; Pauw, L. D.; Toussaint, C.; 1, E. S. G. on T. in H. T. Combined Liver-Kidney and Isolated Liver Transplantations for Primary Hyperoxaluria Type 1: The European Experience. Nephrol. Dial. Transplant. 1991, 6 (7), 502-511.]

It is therefore evident that there is a need to develop new therapies that effectively reduce oxalate levels, are generally applicable in all cases of primary hyperoxalurias and do not involve the risks represented by the aforementioned surgical treatments.

An approach to treatment consisting of the recovery of AGT activity is currently employed. In this regard, one of the approaches that are currently under development is gene therapy using transfection agents capable of incorporating AGT in hepatocytes that are lacking this enzyme. Recently, by using AAV vectors, long-term correction of PH-1 in a mouse model of this disease has been successfully achieved. [(1) Castello, R.; Borzone, R.; D'Aria, S.; Annunziata, P.; Piccolo, P.; Brunetti-Pierri, N. Helper-Dependent Adenoviral Vectors for Liver-Directed Gene Therapy of Primary Hyperoxaluria Type 1. Gene Ther 2016, 23 (2), 129-134. (2) Salido, E.; Rodriguez-Pena, M.; Santana, A.; Beattie, S. G.; Petry, H.; Torres, A. Phenotypic Correction of a Mouse Model for Primary Hyperoxaluria With Adeno-Associated Virus Gene Transfer. Mol Ther 2011, 19 (5), 870-875. (3) Salido, E. C.; Li, X. M.; Lu, Y.; Wang, X.; Santana, A.; Roy-Chowdhury, N.; Torres, A.; Shapiro, L. J.; Roy-Chowdhury, J. Alanine-Glyoxylate Aminotransferase-Deficient Mice, a Model for Primary Hyperoxaluria That Responds to Adenoviral Gene Transfer. Proc. Natl. Acad. Sci. U.S.A. 2006, 103 (48), 18249-18254]. Moreover, the use of pharmacological chaperones is another promising alternative for AGT activity restoration. Pharmacological chaperones are small ligands capable of promoting the correct folding of mutated enzymes. Their use in diseases derived from inborn errors of metabolism is on the rise, and some of these chaperons can already be found on the market [Muntau, A. C.; Leandro, J.; Staudigl, M.; Mayer, F.; Gersting, S. W. Innovative Strategies to Treat Protein Misfolding in Inborn Errors of Metabolism: Pharmacological Chaperones and Proteostasis Regulators. J Inherit Metab Dis 2014, 37 (4), 505-523]. Recently, a study identifying a molecule capable of acting as a pharmacological chaperone for AGT has proven the viability of this technique as a possible treatment for PH-1 [Oppici, E.; Montioli, R.; Dindo, M.; Maccari, L.; Porcari, V.; Lorenzetto, A.; Chellini, S.; Voltattorni, C. B.; Cellini, B. The Chaperoning Activity of Amino-Oxyacetic Acid on Folding-Defective Variants of Human Alanine:Glyoxylate Aminotransferase Causing Primary Hyperoxaluria Type I. ACS Chem. Biol. 2015, 10 (10), 2227-2236].

A different approach in the search for a pharmacological treatment of PH-1 is the strategy embodied in substrate reduction therapy (SRT). This approach can be applied in conditions that are brought about by a loss of an enzyme function [Smid, B. E.; Aerts, J. M. F. G.; Boot, R. G.; Linthorst, G. E.; Hollak, C. E. M. Pharmacological Small Molecules for the Treatment of Lysosomal Storage Disorders. Expert Opin Investig Drugs 2010, 19 (11), 1367-1379], such as PH-1. In these cases, an adverse accumulation of enzyme substrates takes place. SRT is intended for reducing the level of accumulated substrate to a concentration such that it can be metabolised even by residual enzyme activity.

In accordance with this idea, two enzymes are being studied today as targets that have been proven safe for SRT in PH-1. On the one hand, the glycolate oxidase (GO) enzyme catalyses the oxidation of glycolate to glyoxylate (and it also participates in oxidising the latter to oxalate) [Martin-Higueras, C.; Luis-Lima, S.; Salido, E. Glycolate Oxidase Is a Safe and Efficient Target for Substrate Reduction Therapy in a Mouse Model of Primary Hyperoxaluria Type I. Mol Ther 2016, 24(4), 719-725], and on the other hand the proline dehydrogenase (PRODH2 or HYPDH) enzyme exclusively catalyses the first step in the conversion of *trans*-4-hydroxy-L-proline into glyoxylate [Summitt, C. B.; Johnson, L. C.; Jönsson, T. J.; Parsonage, D.; Holmes, R. P.; Lowther, W. T. Proline Dehydrogenase 2 (PRODH2) Is a Hydroxyproline Dehydrogenase (HYPDH) and Molecular Target for Treating Primary Hyperoxaluria. Biochemical Journal 2015, 466 (2), 273-281]. For both enzymes, the existence of healthy individuals who do not have said enzymes has been documented [Frishberg, Y.; Zeharia, A.; Lyakhovetsky, R.; Bargal, R.; Belostotsky, R. J. Med. Genet. 2014, 51(8), 526-529]. Furthermore, for the GO enzyme, knock-out (KO) mice have been generated that have also been developed without any evidence of deleterious phenotypic effects.

PRODH2 inhibition is postulated as being effective for the treatment of the three types of primary hyperoxaluria (PH-1, PH-2 and PH-3), while GO inhibition would be especially useful in the case of PH-1.

Although some small PRODH2 inhibitory molecules have been found, their effectiveness in oxalate reduction, and therefore in the improvement of the PH phenotype, has not been confirmed in cell cultures cell cultures or *in vivo.* On the contrary, the effectiveness of a GO inhibitor (CCPST) in reducing oxalate levels in mice with PH-1 (Agxt1-KO) has recently been confirmed. This study demonstrates pharmacological GO inhibition potential in the development of a treatment for PH-1 in humans and establishes a lead compound for preparing new inhibitors. The literature provides various examples of GO inhibitors that could be co-crystallised with spinach GO (sGO) [Stenberg, K.; Lindqvist, Y. Three-Dimensional Structures of Glycolate Oxidase with Bound Active-Site Inhibitors. Protein Science 1997, 6 (5), 1009-1015] and human GO (hGO) [Murray, M.S.; Holmes, R.P.; Lowther, W.T. Biochem. 2008, 47, 2439-2449].

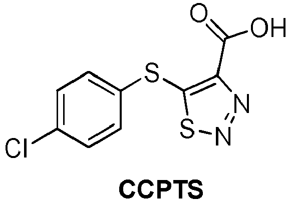

Within the SRT strategy and as an alternative to enzyme inhibition using small molecules, efforts are being made to developing siRNA for GO and PRODH2 [(1) Dutta Chaitali; Salido Eduardo. Inhibition of Glycolate Oxidase with Dicer-Substrate siRNA Reduces Calcium Oxalate Deposition in a Mouse Model of Primary Hyperoxaluria Type I. Molecular therapy: the journal of the American Society of Gene Therapy Journal 2016 (DOI: 10.1038/mt.2016.4). (2) Li, X.; Knight, J.; Fargue, S.; Buchalski, B.; Guan, Z.; Inscho, E. W.; Liebow, A.; Fitzgerald, K.; Querbes, W.; Todd Lowther, W.; et al. Metabolism of 13C5-Hydroxyproline in Mouse Models of Primary Hyperoxaluria and Its Inhibition by RNAi Therapeutics Targeting Liver Glycolate Oxidase and Hydroxyproline Dehydrogenase. Biochimica et Biophysica Acta (BBA) - Molecular Basis of Disease 2016, 1862 (2), 233-239. (3) Querbes, W.; Fitzgerald, K.; Bettencourt, B.; Liebow, A.; Erbe, D. Compositions and Methods for Inhibition of Hao1 (hydroxyacid Oxidase 1 (glycolate Oxidase)) Gene Expression. WO2016057893 (A1), April 14, 2016**].** Studies in Agxt1-KO mice injected with siRNA which targets the silencing of the expression of each of these enzymes have shown a reduction of oxalate excreted in urine, again suggesting a possible treatment for PH-1.

### Derivatives of salicylic acid

Salicylic acid is a natural product of plant origin which has multiple known targets not only in plants but also in animals, including humans. Derivatives of salicylic acid have been used to treat pain, inflammatory processes and fever. Furthermore, there are studies describing the effect of salicylic acid and its derivatives in the treatment of neurodegenerative diseases, hepatitis C, cancer and skin disorders, among others [Klessig, DF, Tian, M and Choi, HW (2016). Multiple Targets of Salicylic Acid and Its Derivatives in Plants and Animals. Front Immunol 7: 206].

There are no references which relate salicylic acid or its derivatives with the capacity to reduce oxalate in AGT-deficient cells, or to the use of this type of compounds, in the treatment of diseases due to oxalate accumulation or, in particular, in the treatment of hyperoxaluria.

There are no known references which relate salicylic acid or its derivatives with GO or PRODH2 inhibitory capacity.

### BRIEF DESCRIPTION OF THE INVENTION

The present invention relates to the use of compounds derived from salicylic acid as agents reducing oxalate excretion and/or inhibiting GO and PRODH2 enzyme activity, and therefore to their application for the treatment of diseases linked to GO and/or PRODH2 action and/or the excess of oxalate. Among these diseases are, inter alia, primary hyperoxalurias (PH-1, PH-2 and PH-3), secondary hyperoxaluria or idiopathic calcium oxalate urolithiasis.

Additionally, the invention relates to the use of derivatives of salicylic acid for the treatment of patients with renal insufficiency (uremia or azotaemia) receiving haemodialysis or peritoneal dialysis, in particular patients treated with ascorbic acid (vitamin C), which is metabolised to oxalate, or patients with fibromyalgia and vulvar pain.

In another aspect, the invention relates to the use of derivatives of salicylic acid; or pharmaceutical compositions comprising one or more of those derivatives, as a medicine, or for manufacturing a medicine, for the treatment of said diseases.

In another aspect, the invention relates to combined preparations comprising derivatives of salicylic acid; or of pharmaceutical compositions comprising them, together with other compounds or drugs used for the treatment of the aforementioned diseases.

In another aspect, the present invention relates to a kit for the preparation of the mentioned compositions or combined preparations.

In a final aspect, the invention also relates to a method for treating said diseases using the mentioned compounds, compositions, combined preparations or kits.

### DETAILED DESCRIPTION OF THE INVENTION

### Definitions

A subject or patient will be understood to mean one *"presenting an excess of oxalate"* when the amount of this compound (oxalic acid salts or esters) in the individual's body, particularly in blood or urine, exceeds normal values due to either an excessive production or else an accumulation of same.

The term *"treatment"* or *"treating"* in the context of this document refers to the administration of a compound or composition according to the invention to improve or eliminate a disease, pathological condition or one or more symptoms associated with said disease or condition in a mammal, preferably in humans. *"Treatment* also covers the improvement or elimination of the physiological sequelae of the disease. Specifically, the concept *"treating"* can be interpreted as:
i. Inhibiting the disease or pathological condition, that is, stopping its development;
ii. Alleviating the disease or pathological condition, that is, causing the regression of the disease or pathological condition;
iii. Stabilising the disease or pathological condition.

Throughout the description and claims, the term *"comprises",* which may also be interpreted as *"consists of",* and its variants do not intend to exclude other technical features, additives, components or steps. For those skilled in the art, other objects, advantages and features of the invention may be inferred from both the description and the embodiment of the invention.

In this context, the present invention addresses the use of derivatives of salicylic acid as GO and/or PRODH2 enzyme activity inhibitors and as agents for reducing the excretion of oxalate, in particular calcium oxalate. This activity renders them useful for their application in the treatment of diseases mediated by GO and/or PRODH2 enzyme activity, in particular diseases linked to an excess of oxalate, such as primary hyperoxalurias (PH-1, PH-2 or PH-3), secondary hyperoxaluria or idiopathic calcium oxalate urolithiasis, inter alia.

The invention also relates to the use of derivatives of salicylic acid for the treatment of patients with renal insufficiency (uremia or azotaemia) receiving haemodialysis or peritoneal dialysis, in particular patients treated with ascorbic acid (vitamin C), which is metabolised to oxalate, or patients with fibromyalgia and vulvar pain.

Throughout the invention, the salts (salicylates) and, by extension, the prodrugs of derivatives of salicylic acid will be understood as included within the term *"derivatives of salicylic acid".*

Thus, a first aspect of the present invention relates to the use of derivatives of salicylic acid for the treatment of diseases or conditions linked to GO and/or PRODH2 enzyme activity, in particular diseases linked to an excess of oxalate, and for the treatment of patients with renal insufficiency (uremia or azotaemia) receiving haemodialysis or peritoneal dialysis, in particular patients treated with ascorbic acid (vitamin C), which is metabolised to oxalate, or patients with fibromyalgia and vulvar pain.

In particular, the invention relates to the use of compounds derived from salicylic acid as a medicine or for preparing a medicine for the treatment of said diseases or conditions.

In a more particular embodiment, the disease or condition mediated with an excess of oxalate is selected from the group consisting of primary hyperoxaluria (PH-1, PH-2 or PH-3), secondary hyperoxaluria and idiopathic calcium oxalate urolithiasis. In a preferred embodiment, the disease or condition is primary hyperoxaluria, more preferably PH-1.

In a preferred embodiment, the derivatives of salicylic acid used are compounds of general structures **I** and **II,** hereinafter also referred to as *"compounds I*" and *"compounds II",* respectively. 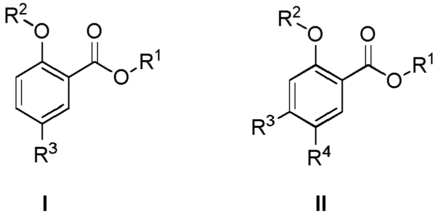 Wherein:
R¹ = -H, -CH₃ and -CH₂CH₃.
R² = -H, - CH₃.
R³ = Aromatic cycle (substituted benzene ring) or aromatic heterocycle (5-membered aromatic heterocycle with oxygen, sulphur or nitrogen), 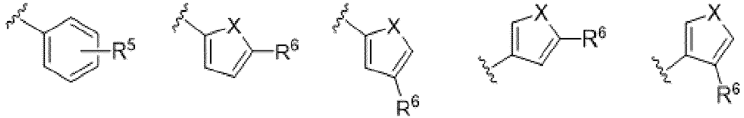
and
R⁴ = -H, -NO₂, -F.
Wherein, in turn:
   R⁵ = Halogen, -NO₂, -O-R⁷, -O-CH₂-R⁷, -_{C}H₂-O-R⁷, -_{C}H₂-O-CH₂-R⁷. R⁶ = -H, -COCH₃, -COR⁷, -COOCH₃, -COOR⁷, -CH₂O⁷, CH₂OCH₃, CH(OH)CH₃, -CH(OH)R⁷, -CH(OH)CH₂R⁷; -CH3, -CHO, -,-CH2NHPh, -CH2-NH-C6H5(4-Br), -CH2-NH-C6H5[4-Bz(4-NHCOCH2CH2CH2CCH)], -CH2-NH-C6H5(4-NO2), -CH2-(piperidine), -CH2-NH-CH2-(3-pyridyl), -CH2NHCH2Ph, - CH2NHCH2CCH,
   R⁷ = H or a substituted aromatic ring with the following structure 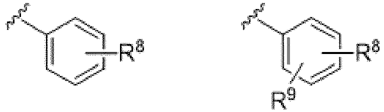
   Wherein, in turn:
      R₈ = -H, -CF₃, halogen, -OCH₃.
      R₉ = -H, halogen.
      and
      X = O, S, N-CH₃.

Structures **I** and **II** satisfy the structural requirements which have been established for GO inhibitors and in fact exhibit the capacity to inhibit said enzyme. The described GO inhibitors are molecules with a polar head (α-hydroxyacid, α-ketoacid, oxamate, sulphonate or heterocyclic) to which hydrophobic aliphatic or aromatic groups are bound. In the literature it is described that the polar head must have a protonated atom in β position with respect to a carboxylate. As has been described, the hydrophobicity of the moieties bound to the polar head play a fundamental role in the inhibitory activity, which increases proportionally with the hydrophobic nature of the side chains.

In the compounds of the invention, the salicylic acid fragment would constitute the polar head of β-hydroxyacid, while the hydrophobic tail would be represented by an apolar moiety which can be an aryl group, a heteroaryl group, an amino group or a halogen.

In a more particular embodiment, the derivatives of salicylic acid used are selected from the subgroups of compounds with general structures **A, B** or **C** (groups selected from compounds **I**), or **D, E,** or **F** (groups selected from compounds **II**), where they are: 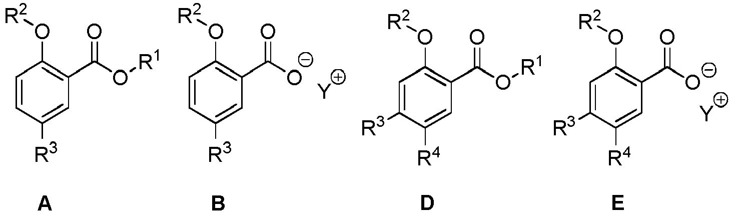 Wherein:
Y = NH4
R¹ = -H and -CH₃.
R² = -H, - CH₃.
R⁴ = -H, -N02, -F
and R³ = Aromatic heterocycle with oxygen, sulphur or nitrogen, according to structures: 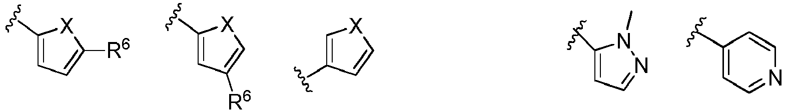 Wherein, in turn,
   X = O, S;
   and
   R6 = -CH3, -CHO, -COCH3 -CH2OH, -CH2NHPh, -CH2-NH-C6H5(4-Br), -CH2-NH-C6H5[4-Bz(4-NHCOCH2CH2CH2CCH)], -CH2-NH-C6H5(4-NO2), -CH2-(piperidine), -CH2-NH-CH2-(3-pyridyl), -CH2NHCH2Ph, -CH2NHCH2CCH, and 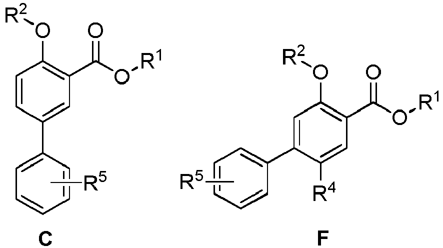
   Wherein:
   R1 = -H, -CH3
   R2 = -H, -CH3
   R4 = -H, -N02, F
   and
   R5 = -N02, -OH, -OCH3, -O-CH2-Ph(4-OCH3), -CH2-O-Ph(3-CF3), -OCH2Ph, -CH2-O-Ph(3,5-F,F), 2-furyl, F, Cl, Br, I.

More particularly, the derivatives of salicylic acid used are selected from the compounds described in detail in Table 1:

**Table 1: Particular selection of derivatives of salicylic acid**

| Subgroup | Compound | R1 | R2 | R3 | R4 | R5 |
|---|---|---|---|---|---|---|
| A | 74 | H | H | 2-furyl | - | - |
| A | 78 | H | H | 5-formyl-2-furyl | - | - |
| A | 76 | H | H | 3-furyl | - | - |
| A | 82 | H | H | 5-hydroxymethyl-2-furyl | - | - |
| A | 86 | H | H | 2-thienyl | | - |
| A | 88 | H | H | 3-thienyl | | - |
| A | 90 | H | H | 5-formyl-2-thienyl | | - |
| A | MDMG-919 | H | H | 5-(piperidinemethyl)-2-furyl | | - |
| A | MDMG-927 | H | H | 5-(3-pyridylmethyl)-2-furyl | | - |
| B | MDMG-907 | NH4 | H | 5-[(4-bromophenyl)aminomethyl]-2-furyl | | - |
| B | MDMG-911 | NH4 | H | 5-{4-[4-(5-hexinamido)benzoyl]phenyl}aminomethyl-2-furyl | | - |
| B | MDMG-915 | NH4 | H | 5-[(4-nitrophenyl)aminomethyl]-2-furyl | | - |
| B | MDMG-931P | NH4 | H | 5-(benzylaminomethyl)-2-furyl | | - |
| B | MDMG-935P | NH4 | H | 5-(propin-1-ylaminomethyl)-2-furyl | | - |
| C | 92 | H | H | - | H | *p*-(4-methoxyphenylmethoxy) |
| C | 94 | H | H | - | H | *o*-[3-(trifluoromethyl)phenyloxymethyl] |
| C | 96 | H | H | - | H | *p*-(benzyloxy) |
| C | 98 | H | H | - | H | *o*-(3,5-difluorophenvioxymethyl) |
| C | MDMG-943 | H | H | - | H | *p*-(2-furyl) |
| D | 73 | H | H | 2-furyl | H | - |
| D | 77 | H | H | 5-formyl-2-furyl | H | - |
| D | 75 | H | H | 3-furyl | H | - |
| D | 79 | CH3 | CH3 | 5-formyl-2-furyl | H | - |
| D | 80 | H | CH3 | 5-formyl-2-furyl | H | - |
| D | 81 | H | H | 5-hydroxymethyl-2-furyl | H | - |
| D | 83 | H | H | 5-phenylaminomethyl-2-furyl | H | - |
| D | 84 | H | H | 2-furyl | NO2 | - |
| D | 85 | H | H | 2-thienyl | H | - |
| D | 87 | H | H | 3-thienyl | H | - |
| D | 89 | H | H | 5-formyl-2-thienyl | H | - |
| D | 302 | H | H | 5-acetyl-2-thienyl | H | - |
| D | 306 | H | H | 5-methyl-2-thienyl | H | - |
| D | 309 | H | H | 1-methyl-1H-pyrazol-5-yl | H | - |
| D | 310 | H | H | 4-pyridyl | H | - |
| F | 91 | H | H | - | H | *p*-(4-methoxyphenylmethoxy) |
| F | 93 | H | H | - | H | *o*-[3-(trifluoromethyl)phenyloxymethyl] |
| F | 95 | H | H | - | H | *p*-(benzyloxy) |
| F | 97 | H | H | - | H | *o*-(3,5-difluorophenyloxymethyl) |
| F | 99 | H | H | - | H | *p*-OH |
| F | 100 | H | H | - | H | *m*-(OCH3) |
| F | 101 | H | H | - | H | *p*-(NO2) |

In a preferred embodiment, the derivatives of salicylic acid are selected from the group consisting of the compounds of general formula **73, 77, 74** and **78** (Table 2).

**Table 2: Preferably used derivatives of salicylic acid.**

| **Compound** | **Structure** | **Compound** | **Structure** |
|---|---|---|---|
| **73** | 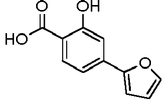 | **74** | 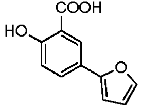 |
| **77** | 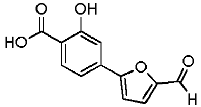 | **78** | 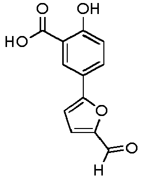 |

### Pharmaceutical compositions

In a second aspect, the invention provides pharmaceutical formulations, forms or compositions, hereinafter *"compositions of the invention"* comprising as an active ingredient a therapeutically effective amount of at least one derivative of salicylic acid for the treatment of the mentioned diseases. Said formulations can contain any other active ingredient in the treatment of patients with the mentioned diseases or can be characterised by containing as an active ingredient only one derivative of salicylic acid or a combination of derivatives of salicylic acid.

In different preferred embodiments, the derivative of salicylic acid is a compound with general structure **I** or **II**, more preferably with general structure **A, B, C, D, E** or **F,** even more preferably one of the compounds described in detail in Table 1 and even more preferably, one of the compounds described in detail in Table 2.

In the sense used in this description, the expression *"therapeutically effective amount"* refers to that amount of a compound which, when administered to a mammal, preferably humans, is sufficient for producing the treatment of diseases mediated by GO and/or PRODH2 enzyme activity, in particular diseases linked to an excess of oxalate, such as PH-1, secondary hyperoxaluria, idiopathic calcium oxalate urolithiasis, among others, or for the treatment of patients with renal insufficiency (uremia or azotaemia) receiving haemodialysis or peritoneal dialysis, in particular patients treated with ascorbic acid (vitamin C), which is metabolised to oxalate, or patients with fibromyalgia and vulvar pain.

The amount of a compound constituting a therapeutically effective amount will vary, for example, according to the activity of the specific compound used; the metabolic stability and duration of the action of the compound; the species (preferably human), the clinical form of the human disease, age, body weight, general state of health, sex and diet of the patient; route of administration, given the possibility of oral or systemic administration; the mode and time of administration; the excretion rate, the combination of drugs; the seriousness of the particular disorder or pathological condition; and the patient being subjected to therapy, but this can be determined by one skilled in the art according to his or her own knowledge and that description.

Moreover, in accordance with the present invention, the *"pharmaceutical form"* is the individual arrangement adapted by drugs (active ingredients) and excipients (pharmacologically inactive material) to form a medicine.

Thus, said pharmaceutical compositions comprise one or more pharmaceutically acceptable carriers.

The *"pharmaceutically acceptable carriers"* that can be used in the formulations of the invention are carriers known to those skilled in the art and normally used in the preparation of therapeutic compositions.

The pharmaceutical composition can optionally comprise another active ingredient. In addition to the therapeutic efficacy requirement, which may necessitate the use of therapeutic agents, in addition to the compounds of the invention, there may be additional fundamental reasons which oblige or recommend to a great extent the use of a combination of a compound of the invention and another therapeutic agent, such as in the treatment of diseases or conditions which directly or indirectly modulate the function of the substance.

The formulations may further contain excipients.

The excipients and carriers used must be pharmaceutically and pharmacologically tolerable, such that they can be combined with other components of the formulation or preparation and have no adverse effects on the treated subject. The pharmaceutical compositions or formulations include those which are suitable for oral or parenteral administration (including subcutaneous, intradermal, intramuscular and intravenous), although the best route of administration depends on the state of the patient. The formulations can be in single dosage form. The formulations are prepared according to methods known in the field of the pharmacology. The amounts of active substances to be administered may vary depending on the particularities of the therapy.

The compositions of the invention are prepared by the usual methods such as those described or referred to in the Spanish and US Pharmacopoeias and similar reference texts.

### Combined preparations

Thus, in another aspect, the invention relates to a composition, preparation or pharmaceutical form, hereinafter *"combined preparation of the invention",* for the treatment of diseases mediated by GO and/or PRODH2 enzyme activity, in particular diseases linked to an excess of oxalate, such as PH-1, secondary hyperoxaluria, idiopathic calcium oxalate urolithiasis, among others; or for the treatment of patients with renal insufficiency (uremia or azotaemia) receiving haemodialysis or peritoneal dialysis, in particular patients treated with ascorbic acid (vitamin C), which is metabolised to oxalate, or patients with fibromyalgia and vulvar pain, which comprises:
a) a derivative of salicylic acid,
b) another active ingredient, including another derivative of salicylic acid different from the aforementioned.

In a particular embodiment, the disease is selected from the group consisting of primary hyperoxalurias (PH-1, PH-2 and PH-3), secondary hyperoxaluria, idiopathic calcium oxalate urolithiasis. In a preferred embodiment, the disease is a primary hyperoxaluria, more preferably PH-1.

In different preferred embodiments, the derivative of salicylic acid is a compound with general structure **I** or **II,** more preferably with general structure **A, B, C, D, E** or **F,** even more preferably one of the compounds described in detail in Table 1 and even more preferably, one of the compounds described in detail in Table 2.

### Kit of the invention

In another aspect, the present invention relates to a kit (*"kit-of-parts"*) for the preparation of the composition or the combined preparation of the invention.

As it is used herein, the term "kit" refers to a combination of a set of components suitable for obtaining the composition or the combined preparation of the invention, which may or may not be packaged together, along with the containers and packages suitable for commercial sale, etc.

In the present invention, *"**component suitable for obtaining the composition or* of *the combined preparation of the invention"* is understood to mean any compound that can be used for obtaining same and includes, without limitation, aqueous solutions, solid preparations, buffers, syrups, preservation solutions, flavourings, pH regulators, thickeners, etc.

The components of the kit can be provided in separate vials (in the form of a "kit-of-parts") or in a single vial. Furthermore, the kit of the present invention is understood to be intended for preparing the composition or combined preparation or pharmaceutical form (for example, the oral solution, mouthwash, rinse, gargle, elixir, etc.) of the invention. Preferably, the components of the kit of the present invention are ready to be used for preparing the composition or combined preparation or pharmaceutical form of the present invention. Furthermore, the kit preferably contains instructions explaining how to prepare the composition or combined preparation or pharmaceutical form of the present invention. The instructions can be provided to users in electronic form or on paper.

Therefore, the invention provides a kit for preparing the composition of the invention or combined preparation of the invention a vessel comprising a container with the compound of the invention in any pharmaceutically acceptable formulation, together with components suitable for obtaining the composition or the combined preparation of the invention.

### Method of treatment of the invention

In another aspect, the present invention relates to a method for treating, hereinafter *"method of treatment of the invention",* patients afflicted with diseases mediated by GO and/or PRODH2 enzyme activity, in particular diseases linked to an excess of oxalate, such as PH-1, secondary hyperoxaluria, idiopathic calcium oxalate urolithiasis, among others; or for the treatment of patients with renal insufficiency (uremia or azotaemia) receiving haemodialysis or peritoneal dialysis, in particular patients treated with ascorbic acid (vitamin C), which is metabolised to oxalate, or patients with fibromyalgia and vulvar pain, by using derivatives of salicylic acid and/or the compositions and/or combined preparations and/or kit of the invention.

In a particular embodiment, the disease is selected from the group consisting of primary hyperoxalurias (PH-1, PH-2 and PH-3), secondary hyperoxaluria, idiopathic calcium oxalate urolithiasis. In a preferred embodiment, the disease is a primary hyperoxaluria, more preferably PH-1.

The effects of this method of treatment include, but are not limited to the effects of the elimination of the disease, the increase in the time of progression of the disease and the survival rate. The long-term effects of the treatment include the control of the disease.

This treatment consists of the administration to individuals afflicted with these diseases of therapeutically effectives amounts of at least one derivative of salicylic acid, or a pharmaceutical composition that include it.

The administration of the derivatives of salicylic acid, in pure form or in a suitable pharmaceutical composition, or in combination with other compounds, compositions or medicines, can be carried out by means of modes of administration of agents accepted for similar uses.

In different preferred embodiments, the derivative of salicylic acid is a compound with general structure **I** or **II**, more preferably with general structure **A, B, C, D, E** or **F,** even more preferably one of the compounds described in detail in Table 1, and even more preferably, one of the compounds described in detail in Table 2.

### EMBODIMENTS

The following examples are provided by way of illustration, and are not intended to limit the present invention. A selection of compounds which have been prepared and/or biologically evaluated are indicated in Table 3. Table 4 further indicates the EC50 found for the reduced production of oxalate in Agxt1-KO mouse hepatocyte cultures.

### Biological evaluation.

### Methods of biological evaluation

*Development of AGXT and GO enzyme-deficient mice:* The AGXT-deficient mice have previously been described. The GO-KO mice were obtained according to the method described in the literature.

*Isolation and culture of hepatocytes*: The isolation of hepatocytes from AGT enzyme-deficient mice was carried out as described in the literature. A total of 3.0 x 10⁵ cells/well were then cultured in 6-well plates in William's E medium supplemented with foetal bovine serum (5 %), I-glutamine (2 mM), penicillin (100 U/ml), streptomycin (100 µg/ml), insulin (2.2 mlU/ml) and hydrocortisone (0.3 µg/ml). After 5 h, the medium was switched to serum-free complete William's E medium (Biochrom, Cambridge, UK), and the cells were treated with increasing concentrations of each of the drugs in the presence of 5 mM glycolate. Samples of the culture medium were collected at 24, 48 and 72 h after treatment, to quantify oxalate.

*Cell viability and cytotoxicity assay*: 1.0 x 10⁴ cells/well were cultured in a 96-well plate. They were treated with the same concentrations of drugs as in the preceding assay. At 24, 48 and 72 h, 20 µl of the *Cell Titer* 96 *Aqueous One Solution* reagent (Promega, Madison, Wisconsin) were added and after incubating at 37 °C for 2 h, absorbance measurements were obtained at 493 nm.

*Determination of oxalate:* The determination of oxalate excreted into the medium was carried out by assaying in the presence of oxalate oxidase using a commercial kit (Trinity Biotech, Co Wicklow, Ireland), following the manufacturer's indications. GraphPad Prism 5 software was used for the graphical representation of the data as the mean ± SD.

The efficacy of reducing oxalate production was measured in primary hepatocyte cultures obtained from hyperoxaluric mice (Agxt1^{-/-}). Cultures of less than 5 days were used to prevent possible interferences due to cell differentiation. For the short-term increase in the production of oxalate in cells and for obtaining detectable levels in standard enzymatic assays, glycolate was added to the culture medium at a concentration of 5 mM. Firstly, the viability of the hepatocytes treated with increasing concentrations of each drug up to 200 µM in the presence of glycolate was tested in comparative assays with control hepatocytes not treated with a drug and at different times.

Oxalate reduction assays were then carried out. Samples of the culture medium were taken every 24 hours after treatment with each of the compounds under evaluation. In each of these samples was measured the concentration of oxalate excreted into the medium by Agxt1^{-/-} hepatocytes. The determination of oxalate in the samples was carried out using a colorimetric enzymatic assay based on two consecutive reactions catalysed by the enzymes oxalate oxidase and peroxidase, respectively. The presence of oxalate gives rise to the formation of a coloured indamines derivative. Possible interferences due to interaction between the compounds under evaluation and the enzymes of the kit were discarded in prior assays on standard oxalate solutions.

The results of these assays can be observed in Tables 3 and 4.

**Table 3: Results obtained for compounds representative. The mean value of relative oxalate at 10 µM ± its standard deviation are shown.**

| **Compound** | **Relative oxalate at 10 µM** |
|---|---|
| 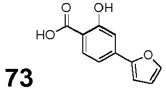 | 0.87 ± 0.15 |
| 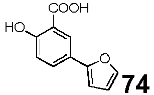 | **-0.25 ± 0.2** |
| 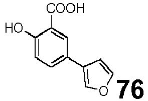 | 0.89 ± 0.16 |
| 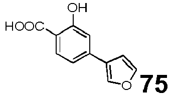 | 1.02 ± 0.3 |
| 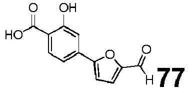 | 0.88 ± 0.18 |
| 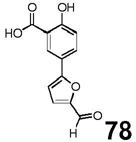 | **-0.06 ± 0.29^{a}** |
| 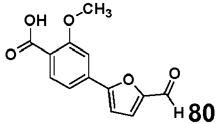 | 0.82 ± 0.04 |
| 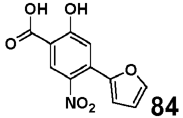 | 0.86 ± 0.04 |
| 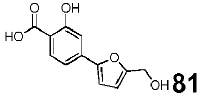 | 0.88 ± 0.01 |
| 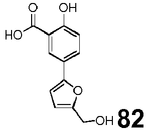 | 0.88 ± 0.19 |
| 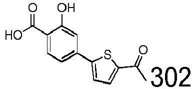 | 0.89 ± 0.14 |
| 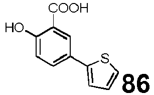 | 0.82 ± 0.01 |
| 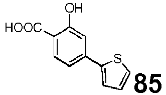 | **0.74 ± 0.16** |
| 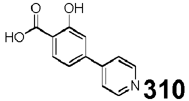 | 0.88 ± 0.03 |
| 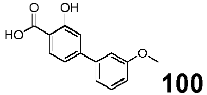 | 0.83 ± 0.03 |
| 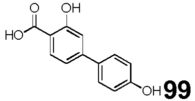 | 0.82 ± 0.01 |
| 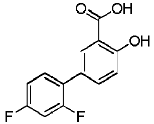 | 0.88 ± 0.08 |
| 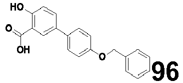 | 0.8 ± 0.16 |
| 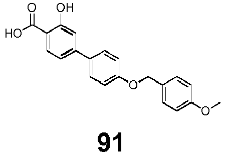 | 0.90 ± 0.16 |
| 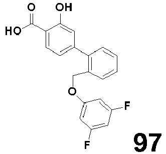 | **0.72** ± 0.16 |
| 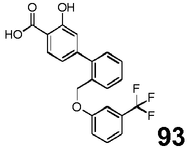 | **0.62 ± 0.29** |
| 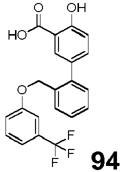 | **0.68 ± 0.21** |
| 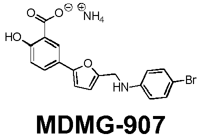 | 0,373 ± 0,063 |
| 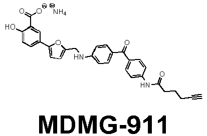 | 0,144 ± 0,322 |
| 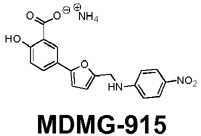 | -0,236 ± 0,115 |
| 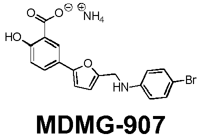 | 0,373 ± 0,063 |

**Table 4. Representative examples of the compounds in respect of which the activity biological that was found has been evaluated. Reference compound: CCPST (EC₅₀(24 h) = 25.26 µM, EC₅₀(48 h) =32.94 µM, EC₅₀ (72 h) = 33.85 µM)**

| **Compound** | **Structure EC₅₀ (µM)** | **Compound** | **Structure EC₅₀ (µM)** |
|---|---|---|---|
| **73** | 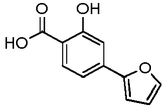 | **74** | 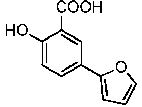 |
| | EC₅₀ (24 h) = 9.35 ± 1.34 | | EC₅₀ (24 h) = 3.59 ± 1.06 |
| | EC₅₀ (48h) = 92.19 ± 1.23 | | EC₅₀ (48 h) = 7.88 ± 1.04 |
| | | | EC₅₀ (72 h) = 9.2 ± 1.04 |
| | | **78** | 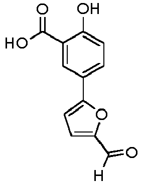 |
| | | | EC₅₀ (24 h) = 3.45 ± 1.17 |
| | | | EC₅₀ (48 h) = 8.36 ± 1.12 |
| | | | EC₅₀ (72 h) = 11.19 ± 1.18 |

### Examples of compound synthesis

*Generalities*: The reagents were commercially obtained and used without purifying. Anhydrous methanol (MeOH) was obtained from commercial sources. Anhydrous dichloromethane (DCM) was obtained by distillation over calcium hydride. The reactions facilitated using microwaves were performed in a Biotage Initiator Microwave with an 8-position arm. The NMR spectra were obtained in 300 MHz instruments (Varian INOVA UNITY), 400 MHz (Varian DIRECT DRIVE) or 500 MHz (Varian DIRECT DRIVE). Chemical shifts (δ) are expressed in ppm and coupling constants (J) in Hz. In the spectra, the abbreviations correspond to singlet (s), broad singlet (bs), doublet (d), broad doublet (bd), double doublet (dd), triple triplet (tt), multiplet (m). The high-resolution mass spectra were recorded in a Waters LCT Premier™ instrument using a time-of-flight (TOF) analyser with electrospray ionisation (ESI) and were measured in positive or negative mode. The reactions were controlled by thin-layer chromatography (TLC) on aluminium plates (Merck Silica gel 60 F254 AL) or by liquid chromatography coupled to a mass spectrometer (LC-MS) in an Agilent 6110 single quadrupole instrument, using a Zorbax Eclipse XDB-C18 4.6 x 150 mm column and electrospray ionisation. Purification by flash chromatography was carried out on silica gel (230-400 mesh ASTM). The purity of the final products was confirmed by HPLC coupled to a diode array detector (Agilent 1200), using a Zorbax Eclipse XDB-C18 4.6 x 150 mm column. The melting points are not corrected.

### Preparation of derivatives of salicylic acid by palladium-catalysed carbon-carbon coupling:

### a) Compounds 73, 87, 91, 92, 93, 95, 99, 100 and 101:

General method: A DMF/H₂O 1/1 mixture is prepared in a microwave vial, in which triphenylphosphine (0.15 equiv), potassium carbonate (3.5 equiv) and palladium acetate (0.05 equiv) are dissolved. Next, the corresponding halosalicylate or halosalicylic acid and boronic acid or boronate are added sequentially. The mixture is then degassed by argon bubbling for 15 min and next the vial was sealed. The reaction is heated in a microwave instrument for organic synthesis at 100 °C for 3 h, maintaining stirring this entire time. After this time lapses, the reaction is left to cool and said reaction is filtered by washing with methanol. The filtrate is concentrated to dryness using a rotavapor, and the resulting residue is purified by flash chromatography (elution with AcOEt: CH₃CN:H₂O:CH₃OH mixtures). The product obtained in the chromatographic separation is dissolved/suspended in acetone and drops of 10 % HCI at pH 1-2 are added, with the formation of a precipitate being observed. The acetone phase is removed and concentrated in a rotavapor. Subsequently, the residue after the evaporation in a rotavapor is dissolved in water and transferred to an Eppendorf tube, where it is centrifuged for 5 min at 13000 rpm. The supernatant is discarded, water is again added to the Eppendorf tube and this operation is repeated 3 more times.

**4-(2-furyl)-2-hydroxybenzoic acid (73):** 2-hydroxy-4-iodobenzoic acid (50 mg, 0.189 mmol), 2-furylboronic acid (38.6 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. Mobile phase for elution in flash chromatography AcOEt: CH₃CN:H₂O:CH₃OH 70:5:2.5:2.5.
Yield after purification: 50 % (25 mg).
¹H NMR (400 MHz, acetone-*d*₆) δ 11.19 (bs, 1H), 7.92 (d, *J* = 8.3 Hz, 1H), 7.72 (m, 1H), 7.31 (dd, *J* = 8.3, 1.6 Hz, 1H), 7.27 (d, *J* = 1.6 Hz, 1H), 7.07 (d, *J* = 3.4 Hz, 1H), 6.61 (dd, *J* = 3.5, 1.8 Hz, 1H).
¹³C NMR (101 MHz, acetone-*d*₆) δ 172.4 (CO), 163.4 (C), 153.2 (C), 144.8 (CH), 138.4 (C), 131.9 (CH), 115.4 (CH), 113.1 (CH), 112.2 (CH), 111.8 (C), 109.4 (CH).
HRMS (TOF, ES⁻): Calculated for C₁₁H₇O₄ (M-H)⁻: m/z 203.0344. 203.0350 found (deviation 3.0 ppm).
m.p. (°C) > 220.

**4-(3-thienyl)-2-hydroxybenzoic acid (87):** 2-hydroxy-4-iodobenzoic acid (50 mg, 0.189 mmol), 3-thienylboronic acid (29.0 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. In this case, prior to chromatographic purification, the residue from evaporating the filtrate was resuspended in acetonitrile. The precipitate was separated from the liquid phase and the latter was discarded. The solid was then purified by flash chromatography (elution with AcOEt: CH₃CN:H₂O:CH₃OH 70:10:5:5 mixture). **87** was obtained in the form of a brown solid.
Yield after purification: 87 % (36 mg).
¹H NMR (500 MHz, methanol-*d*₄) δ 7.78 (dd, *J* = 8.4, 4.0 Hz, 1H), 7.61 (dd, *J* = 2.9, 1.4 Hz, 1H), 7.37 (qd, *J* = 5.1, 2.2 Hz, 2H), 7.09-7.03 (m, 2H).
¹³C NMR (126 MHz, methanol-*d*₄) δ 161.82, 141.51, 141.16, 130.82, 126.08, 125.71, 121.53, 116.41, 113.53.
HRMS (TOF, ES⁻): Calculated for C₁₁H₇O₃S (M-H)⁻: m/z 219.0116. 219.0122 found (deviation 2.7 ppm).
m.p. (°C) > 300.

**4-[4'-(4"-methoxybenzyloxy)phenyl]-2-hydroxybenzoic acid (91):** 2-hydroxy-4-iodobenzoic acid (50 mg, 0.189 mmol), 4-(4'-methoxybenzyloxy)phenylboronic acid (58.6 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. Purification was carried out by flash chromatography (gradient elution using AcOEt: CH₃CN:H₂O:CH₃OH from 70:5:2.5:2.5 to 60:10:10:10 mixture) to obtain compound **91** as a solid. Yield after purification: 17 % (11.3 mg).
¹H NMR (300 MHz, methanol-*d*₄) δ 7.81 - 7.71 (m, 1H), 7.50 - 7.42 (m, 2H), 7.28 (d, *J* = 8.5 Hz, 2H), 6.99 - 6.88 (m, 4H), 6.88 - 6.80 (m, 2H), 4.95 (s, 2H, CH₂), 3.70 (s, 3H, CH₃).
¹³C NMR (151 MHz, methanol-*d*₄) δ 131.8, 130.3, 129.4, 117.6, 116.3, 114.9, 114.8, 70.8 (CH₂), 55.7 (CH₃).
HRMS (TOF, ES⁻): Calculated for C₂₁H₁₇O₅ (M-H)⁻: m/z 349.1076. 349.1071 found (deviation 1.4 ppm).
m.p. (°C) = 199.8.

**5-[4'-(4"-methoxybenzyloxy)phenyl]-2-hydroxybenzoic acid (92):** Methyl 5-iodosalicylate (50 mg, 0,180 mmol), 4-(4'-methoxybenzyloxy)phenylboronic acid(45 mg, 0,216 mmol), PPh₃ (7.1 mg, 0.027 mmol), K₂CO₃ (87.1 mg, 0.63 mmol), Pd(AcO)₂ (2 mg, 0,009 mmol), 1:1 DMF:H₂O (2 ml) were used. In this case, prior to chromatographic purification, the residue from evaporating the filtrate was resuspended in methanol. The precipitate was separated from the liquid phase and the latter was discarded. The solid was then purified by flash chromatography (gradient elution with AcOEt: CH₃CN:H₂O:CH₃OH mixtures from 70:10:5:5 to 60:10:10:10). **92** was obtained in the form of a yellowish solid.
Yield after purification: 20 % (13 mg).
¹H NMR (400 MHz, acetone-*d*₆) δ 8.10 (d, *J* = 2.4 Hz, 1H), 7.80 (dd, *J* = 8.7, 2.5 Hz, 1H), 7.57 (d, *J* = 8.8 Hz, 2H), 7.43 (d, *J* = 8.6 Hz, 2H), 7.09 (d, *J =* 8.8 Hz, 2H), 7.04 (d, *J* = 8.6 Hz, Hz, 1H), 6.96 (d, *J* = 8.7 Hz, 2H), 5.09 (s, 2H), 3.81 (s, 3H).
¹³C NMR (101 MHz, acetone-*d*₆) δ 172.6 (CO), 159.4 (C), 135.0 (CH), 133.2 (C), 132.9 (C), 130.2 (CH), 128.6 (CH), 128.4 (CH), 126.8 (C), 118.6 (CH), 116.2 (CH), 114.7 (CH), 113.3 (C), 70.3 (CH₂), 55.6 (CH₃).
HRMS (TOF, ES⁻): Calculated for C₂₁H₁₇O₅ (M-H)⁻: m/z 349.1076. 349.1084 found (deviation 2.3 ppm).
m.p. (°C) = 177.8.

**4-{2'-[3"-(trifluoromethyl)phenoxymethyl]phenyl}-2-hydroxybenzoic acid (93):** 2-hydroxy-4-iodobenzoic acid (50 mg, 0.189 mmol), 2-[3'-(trifluoromethyl)phenoxymethyl]phenylboronic acid(67.2 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. Purification was carried out by flash chromatography using gradient elution with AcOEt: CH₃CN:H₂O:CH₃OH mixtures from 70:2.5:2.5:1.25 to 70:10:5:5. Compound **93** was obtained as a syrup.
Yield after purification: 79 % (58 mg).
¹H NMR (400 MHz, acetone-*d*₆) δ 7.91 (d, *J* = 8.5 Hz, 1H), 7.68 (m, 1H), 7.51-7.45 (m, 3H), 7.39 (m, 1H), 7.25 (d, *J* = 8.0 Hz, 1H), 7.22-7.18 (m, 2H), 7.03-7.00 (m, 2H), 5.14 (s, 2H).
¹³C NMR (101 MHz, acetone-*d*₆) δ 172.3 (CO), 162.6 (C), 159.8 (C), 149.3 (C), 141.9 (C), 134.5 (C), 132.1 (c, *J_{C-F}* = 32.0 Hz, C), 131.3 (CH), 131.2 (CH), 130.8 (CH), 130.5 (CH), 129.4 (CH), 129.2 (CH), 125.1 (c, *J_{C-F}* = 271.6 Hz, CF₃), 121.1 (CH), 119.6 (CH), 118.5 (CH), 118.3 (c, *J_{C-F}* = 3.9 Hz, CH), 112.4 (c, *J_{C-F}* = 3.9 Hz, CH), 112.1 (C), 69.2 (CH₂).
HRMS (TOF, ES⁻): Calculated for C₂₁H₁₄O₄F₃ (M-H)⁻: m/z 387.0844. 387.0845 found (deviation 0.3 ppm).

**4-[4'-(benzyloxy)phenyl]-2-hydroxybenzoic acid (95):** 2-hydroxy-4-iodobenzoic acid (50 mg, 0.189 mmol), acid 4-(benzyloxy)phenylboronic (51.8 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. In this case, prior to chromatographic purification, the residue from evaporating the filtrate was resuspended in acetonitrile. The precipitate was separated from the liquid phase and the latter was discarded. The solid was then purified by flash chromatography (elution with AcOEt: CH₃CN:H₂O:CH₃OH 60:10:10:10 mixture). **95** was obtained in the form of a white solid.
Yield after purification: 100 % (62 mg).
¹H NMR (400 MHz, acetone-*d*₆) δ 7.93 (d, *J* = 8.3 Hz, 1H), 7.70 (m, 2H), 7.51 (m, 2H), 7.41 (m, 2H), 7.34 (m, 1H), 7.23 (dd, *J* = 8.28, 1.82 Hz, 1H), 7.19 (d, *J* = 1.8 Hz, 1H), 7.14 (m, 2H), 5.21 (s, 2H).
¹³C NMR (101 MHz, acetone-*d*₆) δ 172.5 (CO), 163.3 (C), 160.4 (C), 149.0 (C), 138.2 (C), 132.7 (C), 131.8(CH), 129.3 (CH), 129.2 (CH), 128.7 (CH), 128.5 (CH), 118.3 (CH), 116.2 (CH), 115.2 (CH), 111.4 (C), 70.6 (CH₂).
HRMS (TOF, ES⁻): Calculated for C₂₀H₁₅O₄: (M-H)⁻: m/z 319.0970. 319.0964 found (deviation 1.9 ppm).

**4-(4-hydroxyphenyl)-2-hydroxybenzoic** acid (99): 2-hydroxy-4-iodobenzoic acid (50 mg, 0.189 mmol), 4-hydroxyphenylboronic acid (31.3 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. Purification was carried out by flash chromatography, eluting with an AcOEt: CH₃CN:H₂O:CH₃OH mixture (gradient from 70:5:2.5:2.5 to 60:10:10:10). Compound **99** was obtained in the form of a solid.
Yield after purification: 100 % (44 mg).
¹H NMR (500 MHz, methanol-*d*₄) δ 7.86 (d, *J* = 8.6 Hz, 1H), 7.48 (m, 2H), 7.07-7.05 (m, 2H), 6.89-6.85 (m, 2H).
¹³C NMR (126 MHz, methanol-*d*₄) δ 174.5 (CO), 163.0 (C), 158.9 (C), 148.6 (C), 132.3 (C), 131.9 (CH), 129.2 (CH), 118.0 (CH), 116.6 (CH), 114.9 (CH), 113.8 (C).
HRMS (TOF, ES⁻): Calculated for C₁₃H₉O₄: (M-H)⁻: m/z 229.0501. 229.0510 found (deviation 3.9 ppm).
m.p. (°C) = 265.8.

**4-(3-methoxyphenyl)-2-hydroxybenzoic acid (100):** 2-hydroxy-4-iodobenzoic acid (50 mg, 0.189 mmol), 3-methoxyphenylboronic acid (34.5 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. In this case, prior to chromatographic purification, the residue from evaporating the filtrate was resuspended in acetonitrile. The precipitate was separated from the liquid phase and the latter was discarded. The solid was then purified by flash chromatography (gradient elution with AcOEt: CH₃CN:H₂O:CH₃OH mixture, 70:5:2.5:2.5 to 60:10:10:10). **100** was obtained in the form of a brown solid.
Yield after purification: 86 % (25 mg).
¹H NMR (500 MHz, methanol-*d*₄) δ 7.92 (d, *J* = 8.0 Hz, 1H), 7.33 (t, *J* = 7.9 Hz, 1H), 7.19-7.16 (m, 1H), 7.13 (m, 1H), 7.09 (bs, 1H), 7.08 (m, 1H), 6.92 (dd, *J* = 8.3, 2.6 Hz, 1H), 3.82 (s, 3H, OCH₃).
¹³C NMR (126 MHz, methanol-*d*₄) δ 174.7 (CO), 163.0 (C), 161.4 (C), 148.3 (C), 142.8 (C), 132.2 (CH), 130.9 (CH), 120.4 (CH), 118.5 (CH), 115.8 (CH), 115.7 (C), 114.5 (CH), 113.7 (CH), 55.7 (CH₃).
HRMS (TOF, ES⁻): Calculated for C₁₄H₁₁O₄: (M-H)⁻: m/z 243.0657. 243.0659 found (deviation 0.8 ppm).
m.p. (°C): 185.1.

**4-(4-nitrophenyl)-2-hydroxybenzoic acid (101):** 2-hydroxy-4-iodobenzoic acid (50 mg, 0.189 mmol), 4-nitrobenzeneboronic acid (38 mg, 0.227 mmol), PPh₃ (7.4 mg, 0.028 mmol), K₂CO₃ (91.4 mg, 0.662 mmol), Pd(AcO)₂ (2.12 mg, 0.0095 mmol), 1:1 DMF:H₂O (2 ml) were used. In this case, prior to chromatographic purification, the residue from evaporating the filtrate was resuspended in acetonitrile. The precipitate was separated from the liquid phase and the latter was discarded. The solid was then purified by flash chromatography (elution with AcOEt: CH₃CN:H₂O:CH₃OH 70:10:5:5 mixture). Compound **101** was obtained as a yellowish solid.
Yield after purification: 69.5 % (34 mg).
¹H NMR (500 MHz, methanol-*d*₄) δ 8.31 (d, *J* = 8.9 Hz, 2H), 7.96 (d, *J* = 7.94 Hz, 1H), 7.87 (d, *J* = 8.9 Hz, 2H), 7.17-7.13 (m, 2H).
¹³C NMR (126 MHz, methanol-*d*₄) δ 175.2 (CO), 163.1 (C), 148.7 (C), 148.2 (C), 144.5 (C), 132.4 (CH), 129.1 (2CH), 124.9 (2CH), 119.5 (C), 118.1 (CH), 116.1 (CH).
HRMS (TOF, ES⁻): Calculated for C₁₃HₛNO₅: (M-H)⁻: m/z 258.0402. 258.0411 found (deviation 3.5 ppm).
m.p. (°C) > 300

### b) 5-(2-furyl)-2-hydroxybenzoic acid (74):

A solution of potassium carbonate (74.63 mg, 0.540 mmol) in water (1 ml) was prepared in a microwave vial and 0.5 ml of DMF was added to it. Next, methyl 2-hydroxy-5-iodobenzoate (50 mg, 0,180 mmol), 2-furanboronic acid (24.2 mg, 0,216 mmol), PPh₃ (7.1 mg, 0,027 mmol), Pd(OAc)₂ (2.02 mg, 0.009 mmol) and 0.5 ml of DMF were added. The mixture was degassed by argon bubbling for 10 min and the vial was closed. The reaction was programmed in a microwave instrument for organic synthesis at 100 °C for 3 h. Once the reaction ended, it was filtered by washing with MeOH to eliminate the impurities and the filtrate was concentrated in the rotavapor. The residue was acidified with 10 % HCI and purified by flash chromatography
(mobile phase AcOEt/CH₃CN/MeOH/H₂O 70:10:5:5). Compound **74** was obtained as a brown solid.
Yield after purification: 83 % (30 mg).
¹H NMR (400 MHz, methanol-*d*₄) δ 8.18 (s, 1H), 7.65 (d, *J* = 7.9 Hz, 1H), 7.48-7.41 (s, 1H), 6.85 (d, *J* = 8.5 Hz, 1H), 6.55 (d, *J* = 3.3 Hz, 1H), 6.43 (dd, *J* = 1.8, 3.4 Hz, 1H).
HRMS (TOF, ES⁻): Calculated for C₁₁H₇O₄ (M-H)⁻: m/z 203.0344. 203.0350 found (deviation 3.0 ppm).

### c) Compounds 75, 76, 85, 86 and 88:

General method: A solution of potassium carbonate (3 equiv) in water (2 ml/mmol of halosalicylate compound) is prepared in a sealed tube and an equal volume of DMF is added to it. Next, halosalicylic acid or halosalicylate (1 equiv), boronic acid or boronate (1.2 equiv), triphenylphosphine (0.15 equiv) and palladium acetate (0.05 equiv) compounds are added. The mixture is degassed by argon bubbling for 10 min and the tube is closed. It is left to react in an oil bath at 100 °C for 24 h. Once the reaction has ended, it is concentrated in the rotavapor, resuspended in acetonitrile and filtered. The solid is resuspended in water and acidified with 5 % HCl. The solvent is evaporated and the obtained residue is purified by flash chromatography.

**4-(3-furyl)-2-hydroxybenzoic acid (75):** 60 mg of 2-hydroxy-4-iodobenzoic acid were used (0.227 mmol), 52.77 mg of 2-pinacolyl furanboronate (0.272 mmol), 94.12 mg of K₂CO₃ (0.681 mmol), 8.92 mg of PPh₃ (0.034 mmol), 2.47 mg of Pd(OAc)₂ (0.011 mmol), 1:1 DMF:H₂O (2 ml) were used. The obtained crude reaction product was purified by flash column chromatography using as the mobile phase DCM/MeOH (20:1 →9:1 gradient) followed by AcOEt/CH₃CN/MeOH/H₂O (70:5:2.5:2.5→ 70:2.5:1.25:1.25 gradient). Compound 75 was obtained as a brown solid.
Yield after purification: 47 % (21 mg).
Melting point >300 °C
¹H NMR (400 MHz, acetone-*d*₆) δ 8.14 (s, 1H), 7.93 (d, *J* = 6.8 Hz, 1H), 7.66 (s, 1H), 7.14 (s, 2H), 6.93 (s, 1H).
¹³C NMR (101 MHz, acetone-*d*₆) δ 166.92, 162.56, 144.67, 140.88, 139.61, 131.63, 126.02, 116.68, 113.74, 110.38, 108.92.
HRMS (TOF, ES⁻): Calculated for C₁₁H₇O₄ (M-H)⁻: m/z 203.0344. 203.0347 found (deviation 1.5 ppm).

**5-(3-furyl)-2-hydroxybenzoic acid (76):** 60 mg of methyl 2-hydroxy-5-iodobenzoate (0.216 mmol), 50.25 mg of 3-pinacolyl furanboronate (0.259 mmol), 89.56 mg of K₂CO₃ (0.648 mmol), 8.39 mg of PPh₃ (0.032 mmol), 2.47 mg of Pd(OAc)₂ (0.011 mmol), 1:1 DMF:H₂O (2 ml) were used. The obtained crude reaction product was purified by flash column chromatography using as the mobile phase DCM/MeOH (20:1 →9:1 gradient) followed by AcOEt/CH₃CN/MeOH/H₂O (70:5:2.5:2.5→70:2.5:1.25:1.25 gradient). Compound **76** was obtained as a brown solid.
Yield after purification: 34 % (15 mg).
Melting point: >300 °C
¹H NMR (500 MHz, methanol-*d*₄) δ 8.03 (s, 1H), 7.8 (s, 1H), 7.55 (dd, *J* = 8.5, 1.8 Hz, 1H), 7.51 (t, *J* = 1.7 Hz, 1H), 6.87 (d, *J* = 8.5 Hz, 1H), 6.73 (d, *J* = 1.8 Hz, 1H).
¹³C NMR (126 MHz, methanol-*d*₄) δ 161.8 (C), 144.9 (CH), 144.5 (C), 139.0 (CH), 138.6 (C), 132.4 (CH), 128.7 (CH), 127.3 (C), 124.4 (C), 118.0 (CH), 109.5 (CH).
HRMS (TOF, ES⁻): Calculated for C₁₁H₇O₄ (M-H)⁻: m/z 203.0344. 203.0345 found (deviation 0.5 ppm).

**2-hydroxy-4-(2-thienyl)benzoic acid (85):** 70 mg of 2-hydroxy-4-iodobenzoic acid (0.265 mmol), 40.69 mg of 2-thienylboronic acid (0.318 mmol), 109.89 mg of K₂CO₃ (0.795 mmol), 10.49 mg of PPh₃ (0.040 mmol), 2.91 mg of Pd(OAc)₂ (0.013 mmol), 1:1 DMF:H₂O (2 ml) were used. Purification by flash chromatography was performed using as the mobile phase DCM/MeOH (20:1 →9:1 gradient elution). Compound 85 was obtained as a yellow solid.
Yield after purification: 51 % (30 mg).
Melting point = 225 °C
¹H NMR (400 MHz, DMSO-*d*₆) δ 7.74 (d, *J* = 8.4 Hz, 1H), 7.57 (dd, *J* = 5.1, 1.1 Hz, 1H), 7.54 (dd, *J* = 3.6, 1.2 Hz, 1H), 7.13 (dd, *J* = 5.1, 3.7 Hz, 1H), 7.05-6.99 (m, 2H).
¹³C NMR (101 MHz, DMSO-*d*₆) δ 172.01 (CO), 162.95 (C), 143.33 (C), 138.30 (C), 131.33 (CH), 128.50 (CH), 126.72 (CH), 124.89 (CH), 117.40 (C), 114.95 (CH), 112.94 (CH).
HRMS (TOF, ES⁻): Calculated for C₁₁H₇O₃S (M-H)⁻: m/z 219.0116. 219.0105 found (deviation -5.0 ppm).

**2-hydroxy-5-(2-thienyl)benzoic acid (86):** 60 mg of methyl 2-hydroxy-5-iodobenzoate (0.216 mmol), 33.14 mg of 2-thienylboronic acid (0.259 mmol), 89.56 mg of K₂CO₃ (0.648 mmol), 8.40 mg of PPh₃ (0.032 mmol), 2.47 mg of Pd(OAc)₂ (0.011 mmol), 1:1 DMF:H₂O (2 ml) were used. Purification by flash chromatography was performed using as the mobile phase DCM/MeOH (20:1→9:1 gradient elution) followed by AcOEt/CH₃CN/MeOH/H₂O (70:5:2.5:2.5→70:2.5:1.25:1.25 gradient elution). Compound **86** was obtained as a yellow solid.
Yield after purification: 64 % (30 mg).
Melting point > 300 °C
¹H NMR (400 MHz, acetone-*d*₆) δ 8.19 (s, 1H), 7.79 (d, *J* = 6.5 Hz, 1H), 7.37 (s, 2H), 7.07 (s, 1H), 6.99 (d, *J* = 8.4 Hz, 1H).
¹³C NMR (101 MHz, acetone-*d*₆) δ 172.9 (CO), 162.0 (C), 143.8 (C), 133.4 (CH), 128.9 (CH), 128.1 (CH), 126.4 (C), 124.9 (CH), 123.3 (CH), 118.5 (CH). HRMS (TOF, ES⁻): Calculated for C₁₁H₇O₃S (M-H)⁻: m/z 219.0116. 219.0100 found (deviation -7.3 ppm).

**2-hydroxy-5-(3-thienyl)benzoic acid (88):** 70 mg of methyl 2-hydroxy-5-iodobenzoate (0.252 mmol), 38.69 mg of 3-thienylboronic acid (0.302 mmol), 104.49 mg of K₂CO₃ (0.756 mmol), 9.97 mg of PPh₃ (0.038 mmol), 2.92 mg of Pd(OAc)₂ (0.013 mmol), 1:1 DMF:H₂O (2 ml) were used. Purification by flash chromatography was performed using as the mobile phase DCM/MeOH (20:→9:1 gradient elution) followed by AcOEt/CH₃CN/MeOH/H₂O (70:5:2.5:2.5→70:2.5:1.25:1.25 gradient elution). Compound **88** was obtained as a brown solid.
Yield after purification: 72 % (40 mg).
Melting point = 222 °C
¹H NMR (400 MHz, acetone-*d*₆) δ 11.04 (s, 1H), 8.15 (d, *J* = 2.4 Hz, 1H), 7.84 (dd, *J* = 8.6, 2.3 Hz, 1H), 7.67 - 7.61 (m, 1H), 7.51 (dd, *J* = 5.0, 2.9 Hz, 1H), 7.48 - 7.43 (m, 1H), 6.98 (d, *J* = 8.7 Hz, 1H).
¹³C NMR (101 MHz, acetone-*d*₆) δ 172.4, 162.0, 141.6, 134.6, 128.3, 128.1, 127.3, 126.6, 120.3, 118.5, 113.1.
HRMS (TOF, ES⁻): Calculated for C₁₁H₇O₃S (M-H)⁻: m/z 219.0116. 219.0104 found (deviation -5.5 ppm).

### d) Methyl 4-(5-formyl-2-furyl)-2-methoxybenzoate (79).

142.2 mg of K₂CO₃ (1.029 mmol) were dissolved in a flask in 1 ml of H₂O and 0.5 ml of DMF were added. Next, 60 mg of 5-bromofuraldehyde (0.343 mmol), 86.31 mg of 3-methoxy-4-methoxycarbonylbenzene boronic acid (0.411 mmol), 13.37 mg of PPh₃ (0.051 mmol), 3.82 mg of Pd(OAc)₂ (0.017 mmol) and 0.5 ml of DMF were added. The mixture was degassed by argon bubbling for 10 min and was reacted for 1 h at 80 °C. Once the reaction ended, it was concentrated in a rotavapor and the obtained crude reaction product was purified by flash column chromatography using as the mobile phase AcOEt/Hexane (1:4→1:2 gradient elution).
Yield after purification: 57 % (51 mg).
m.p.: 126 °C
¹H NMR (400 MHz, CDCl₃) δ 9.69 (s, 1H), 7.86 (d, *J* = 8.0 Hz, 1H), 7.41 (s, 1H), 7.39 (dd, *J* = 8.1, 1.5 Hz, 1H), 7.34 (d, *J* = 3.8 Hz, 1H), 6.94 (d, *J* = 3.7 Hz, 1H), 4.00 (s, 3H), 3.91 (s, 3H).
¹³C NMR (101 MHz, CDCl₃) δ 177.53, 166.16, 159.78, 158.01, 152.60, 133.68, 132.55, 123.35, 120.87, 117.24, 109.58, 108.47, 56.45, 52.32.
HRMS (TOF, ES⁺): Calculated for C₁₄H₁₂N₂O₅Na (M+Na)⁺:(m/z) 283.0582. 283.0591 found (deviation 3.2 ppm).

### e) 2-hydroxy-4-(5-formyl-2-thienyl)benzoic acid (89):

A solution with 110 mg of K₂CO₃ (0.795 mmol) in 1 ml of H₂O was prepared in a sealed tube and 0.5 ml of DMF were added to it. Next, 70 mg of 2-hydroxy-4-iodobenzoic acid (0.265 mmol), 40.62 mg of 5-formyl-2-thienylboronic acid (0.318 mmol), 10.49 mg of PPh₃ (0.040 mmol), 2.91 mg of Pd(OAc)₂ (0.013 mmol) and 0.5 ml of DMF were added. The mixture was degassed by argon bubbling for 10 min and the tube was sealed. It was then left to react in a bath at 100 °C for 24 h. Once the reaction ended, it was concentrated in the rotavapor, the residue was resuspended in MeOH, and the resulting solid was washed with methanol and acetonitrile. The final solid was resuspended in water and acidified with 5 % HCl. Purification of the resulting evaporation residue was performed by flash chromatography using as the mobile phase DCM/MeOH (gradient 20:1 →9:1). Compound **89** was obtained as a brown solid.
Yield after purification: 56 % (30 mg).
Melting point = 250 °C.
¹H NMR (400 MHz, DMSO-*d*₆) δ 9.91 (s, 1H), 8.03 (d, *J =* 4.0 Hz, 1H), 7.79 (t, *J* = 6.2 Hz, 2H), 7.21 (d, *J* = 1.6 Hz, 1H), 7.19 (dd, *J* = 8.0, 1.7 Hz, 1H).
¹³C NMR (101 MHz, DMSO-*d*₆) δ 184.19, 171.08, 162.45, 151.77, 142.46, 139.09, 136.90, 131.14, 126.20, 117.58, 115.53, 113.69.
HRMS (TOF, ES⁻): Calculated for C₁₂H₇O₄S (M-H)⁻: m/z 247.0065. 247.0060 found (deviation -2.0 ppm).

### f) Compounds 97 and 98:

General method: A solution of potassium carbonate (4 equiv) in water which was previous degassed by argon bubbling (15 min) is prepared in a closed tube. Argon bubbling is maintained for another 5 min, after which time dimethylformamide (DMF) is added (at a 1:1 ratio with respect to water). The corresponding halosalicylic acid or halosalicylate (1 equiv), boronic acid (1.2 equiv), triphenylphosphine (0.12 equiv) and palladium acetate (0.04 equiv) are added to the mixture, in that order. Next, the reaction is heated at 100 °C (oil bath) for 24 h. After said time, the reaction is stopped by cooling and the solvents are evaporated in a rotavapor. The residue is resuspended in 5 % HCI, is filtered, and the resulting solid is washed with small volumes of acetonitrile. It is finally purified by flash chromatography.

**4-[2'-(3",5"-Difluorophenoxymethyl)phenyl]-2-hydroxybenzoic acid (97):** 2-hydroxy-4-iodobenzoic acid (50 mg, 0.19 mmol), acid 2-[(3',5'-difluorophenoxy)methyl]phenylboronic (60 mg, 1.2 mmol), K₂CO₃ (104 mg, 0.76 mmol), Pd(AcO)₂ (2.1 mg, 0.01 mmol), PPh₃ (7.45 mg, 0.03 mmol) and 1:1 DMF:H₂O (2 ml) were used. Column purification was carried out by gradient elution (MeOH:AcOEt mixture from 1:10 to 1:9). Compound **97** was obtained as a white solid.
Yield after purification: 65 % (44 mg).
¹H NMR (500 MHz, DMSO-*d*₆) δ 7.77 (d, *J* = 3.4 Hz, 1H), 7.57 (dd, *J* = 7.5, 1.6 Hz, 1H), 7.46-7.38 (m, 3H), 7.32 (dd, *J* = 7.6, 1.5 Hz, 1H), 6.86 (d, *J* = 8.4 Hz, 1H), 6.75 (tt, *J* = 9.4, 2.3 Hz, 1H), 6.70-6.65 (m, 2H), 4.93 (s, 2H).
¹³C NMR (101 MHz, acetone-*d*₆) δ 175.2 (CO), 164.5 (dd, *J_{C-F}* = 244.6, 16 Hz, 2C-F), 162.1 (C), 161.7 (t, *J_{C-F}* = 13.9 Hz, C), 146.6 (C), 142.5 (C), 134.1(C), 132.4 (CH), 130.6 (CH), 130.3 (CH), 129.2 (CH), 128.7 (CH), 120.0 (CH), 117.9 (CH), 117.3 (C), 99.6 (dd, *J_{C-F}* = 20.5, 7.9 Hz, 2-CH), 96.8 (t, *J_{C-F}* = 26.3 Hz, CH), 69.5 (CH₂).
¹⁹F NMR (376 MHz, DMSO-*d*₆) -109.35 (m, 2F).
HRMS (TOF, ES⁻): Calculated for C₂₀H₁₃O₄F₂ (M-H)⁻: m/z 355.0782. 355.0796 found (deviation 3.9 ppm).

**5-[2'-(3",5"-Difluorophenoxymethyl)phenyl]-2-hydroxybenzoic acid (98):** Methyl 2-hydroxy-4-iodobenzoate (50 mg, 0.19 mmol), acid 2-[(3',5'-difluorophenoxy)methyl]phenylboronic (57 mg, 0.21 mmol), K₂CO₃ (99 mg, 0.72 mmol), Pd(AcO)₂ (0.2 mg, 0.01 mmol), PPh₃ (7.10 mg, 0.27 mmol) and 1:1 DMF:H₂O (2 ml) were used. Column purification was carried out by gradient elution (MeOH:AcOEt mixture from 1:10 to 1:9). Compound **98** was obtained in the form of a white solid.
Yield after purification: 62 % (40 mg).
¹H NMR (500 MHz, DMSO-*d*₆) δ 7.70 (d, *J* = 7.8 Hz, 1H), 7.56 (dd, *J* = 7.0, 3.0 Hz, 1H), 7.46-7.39 (m, 2H), 7.33 (m, 1H), 6.75 (tt, *J* = 9.4, 2.3 Hz, 1H), 6.70-6.64 (m, 4H), 4.97 (s, 2H).
¹³C NMR (101 MHz, acetone-*d*₆) δ 183.1 (CO), 164.5 (dd, *J_{C-F}* = 244.5, 16 Hz, 2C-F), 162.1 (C), 161.7 (t, *J_{C-F}* = 13.8 Hz, C), 142.4 (C), 141.4 (C), 136.9 (C), 134.3 (C), 131.8 (CH), 131.1 (CH), 129.5 (CH), 128.4 (CH), 117.8 (CH), 99.5 (dd, *J_{C-F}* = 20.3, 8.0 Hz, 2-CH), 96.8 (t, *J_{C-F}* = 26.3 Hz, CH), 69.8 (CH₂).
¹⁹F NMR (376 MHz, DMSO-*d*₆) -109.29 (m, 2F).
HRMS (TOF, ES⁻): Calculated for C₂₀H₁₃O₄F₂ (M-H)⁻: m/z 355.0782. 355.0796 found (deviation 3.9 ppm).

### 4-(5-formyl-2-furyl)-2-methoxybenzoic acid (80):

22 mg of **79** (0.084 mmol) were dissolved in a flask in 0.42 ml of THF and 0.42 ml of a solution of 1N NaOH were added. It was left to react for 2 h at 60 °C. Once the reaction ended, it was concentrated in a rotavapor to eliminate THF. 10 ml of H₂O were added and it was acidified with 10 % HCI, whereby an orange-coloured product precipitated. It was filtered by washing with H₂O and the obtained solid was collected. After concentrating in a rotavapor, the obtained crude product was purified by flash column chromatography using as the mobile phase 20:1→9:1 DCM/MeOH and 70:10:5:5 AcOEt/CH₃CN/MeOH/H₂O. **80** was obtained as an orange-coloured solid.
Yield after purification: 30 % (6 mg).
¹H NMR (300 MHz, (CD₃)₂CO) δ 9.72 (s, 1H), 7.99 (d, *J* = 8.3 Hz, 1H), 7.67 (s, 1H), 7.65-7.55 (m, 2H), 7.38 (s, 1H), 4.11 (s, 3H).
HRMS (TOF, ES⁻): Calculated for C₁₃H₉O₅ (M-H)⁻:(m/z) 245.0450. 245.0436 found (deviation -5.7 ppm).

### 4-(5-hydroxymethyl-2-furyl)-2-hydroxybenzoic acid (81):

In round-bottom flask, a solution of 4-(5-formyl-2-furyl)-2-hydroxybenzoic acid (**77**) (33 mg, 0.142 mmol) in methanol (2-3 ml) was prepared and cooled at 0 °C using an ice bath. Next, sodium borohydride (10.8 mg, 0.284 mmol) was slowly added and the reaction was kept under stirring at room temperature until the complete disappearance of the starting product (TLC monitoring) (1 h). The reaction was then acidified to pH 5.0 by adding HCI (5 %), and the reaction was then filtered. The filtrate was concentrated in a rotavapor, and the residue was purified by flash chromatography (elution with AcOEt: CH₃CN: H₂O: CH₃OH mixture in a gradient from 70:5:2.5:2.5 to 60:10:10:10) to obtain compound **81** as a yellowish solid.
Yield after purification: 37.5 % (12.5 mg).
¹H NMR (400 MHz, methanol-*d*₄) δ 7.86 (d, *J* = 8.5 Hz, 1H), 7.16-7.09 (m, 2H), 6.76 (d, *J* = 3.3 Hz, 1H), 6.40 (d, *J* = 3.3 Hz, 1H), 4.58 (s, CH₂, 2H).
HRMS (TOF, ES⁻) Calculated for C₁₂H₉O₅: (M-H)⁻: m/z 233.0450. 233.0450 found (deviation 0 ppm).
m.p. (°C) > 300.

### 5-(5-hydroxymethyl-2-furyl)-2-hydroxybenzoic acid (82):

In round-bottom flask, a solution of 5-(5-formyl-2-furyl)-2-hydroxybenzoic acid (**78**) (40 mg, 0.172 mmol) in methanol (2-3 ml) was prepared and cooled at 0 °C using an ice bath. Next, sodium borohydride (13.0 mg, 0.344 mmol) was slowly added, and the reaction was kept under stirring at room temperature until the complete disappearance of the starting product (TLC monitoring) (3.5 h). The reaction was then acidified to pH 5.0 by adding HCI (5 %), and the reaction was then filtered. The filtrate was concentrated in a rotavapor, and the crude product was purified by flash chromatography (elution with AcOEt: CH₃CN: H₂O: CH₃OH 60:10:10:10 mixture). **82** (25.6 mg, 63.6 %) was thus obtained as a yellowish solid.
¹H NMR (400 MHz, methanol-*d*₄) δ 8.32 - 7.90 (m, 1H), 7.73 (dd, *J* = 8.7, 2.3 Hz, 1H), 6.92 (d, *J* = 8.7 Hz, 1H), 6.54 (d, *J* = 3.3 Hz, 1H), 6.20 (dd, *J* = 3.3, 0.9 Hz, 1H), 4.10 (s, 2H).
¹³C NMR (101 MHz, methanol-*d*₄) δ 171.82 (CO), 160.81 (Cq), 152.02 (Cq), 150.83 (Cq), 134.34 (Cq), 130.41 (arom CH), 124.72 (arom CH), 122.70 (Cq), 117.26 (arom CH), 108.21 (arom CH), 104.37 (arom CH), 26.78 (CH₂).
HRMS (TOF, ES⁻) Calculated for C₁₂H₉O_{S} (M-H)⁻: 233.0450. 233.0442 found.
m.p. (°C): > 300.

### 4-(5-phenylaminomethyl-2-furyl)-2-hydroxybenzoic acid (83):

A solution of 4-(5-formyl-2-furyl)-2-hydroxybenzoic acid (**77**) (30 mg, 0.129 mmol) was prepared in an anhydrous 1:1 mixture of methanol:dichloromethane (4 ml). Next, an activated molecular sieve and aniline (17.7 □ L, 0.194 mmol) were added, and the reaction was kept under stirring, protected from light and at room temperature for 1 h. The disappearance in that time of the starting compound was confirmed by TLC. The reaction flask was then cooled at 0 °C for the addition of sodium triacetoxyborohydride (68.3 mg, 0.323 mmol) in a single portion. The reaction was left to reach room temperature, after which it was kept under stirring for 2 h. Next, the molecular sieve was removed by filtration, and the mixture was acidified to pH 5.0 with HCI (5 %). The solvent was then evaporated in a rotavapor, and the crude product was purified by flash chromatography (gradient elution with CH₂Cl₂: CH₃OH mixtures from 20:1 to 6:1) to obtain **83** as a solid.
Yield after purification: 15 % (6.1 mg).
HRMS (TOF, ES⁻) Calculated for C₁₈H₁₄NO₄ (M-H)⁻: 308.0923. 308.0922 found.
m.p. (°C) > 300.

### 4-L2-furyl)-2-hydroxy-5-nitrobenzoic acid (84):

2-hydroxy-4-iodo-5-nitrobenzoic acid (**103**): 30 mg of 2-hydroxy-4-iodobenzoic acid (0.114 mmol) were dissolved in 3 ml of acetic acid. In cold conditions, 8 µl of HNO₃ (60 %) (0.114 mmol) and 12 µl of H₂SO₄ (95-97 %) (0.227 mmol) were added. It was left to react at room temperature for 48 h. After that time, the reaction was concentrated and purified by flash column chromatography using as the mobile phase CH₂Cl₂-(CH₃)₂CO (2:1→1:1 gradient), (CH₃)₂CO and AcOEt/ACN/MeOH/H₂O (6:2:2:2). Compound **84** was obtained as a yellow solid.
Yield after purification: 74 % (26 mg).
¹H NMR (300 MHz, acetone-*d*₆) δ 8.51 (s, 1H), 7.41 (s, 1H).
HRMS (TOF, ES⁻): Calculated for C₇H₃NO₅I (M-H)⁻: m/z 307.9076. 307.9076 found (deviation 6.5 ppm).

4-(2-furyl)-2-hydroxy-5-nitrobenzoic acid (**84**): 55.15 mg of K₂CO₃ (0.399 mmol) were dissolved in a sealed tube in 1 ml of H₂O, and 0.5 ml of DMF were added. Next, 41 mg of compound **103** (0.132 mmol), 17.79 mg of 2-pinacolyl furanboronate (0.159 mmol), 4.98 mg of PPh₃ (0.019 mmol), 1.34 mg of Pd(OAc)₂ (0.006 mmol) and 0.5 ml of DMF were added. The mixture was degassed by argon bubbling for 10 min, and the tube was sealed. It was left to react in a bath at 100 °C for 32 h. Once the reaction ended, it was concentrated in the rotavapor. The residue was dissolved in H₂O and acidified with 5 % HCI. The obtained crude reaction product was purified by flash column chromatography using as the mobile phase DCM/MeOH (15:1 →9:1 gradient) followed by AcOEt/CH₃CN/MeOH/H₂O (70:5:2.5:2.5→70:10:10:10 gradient). Compound **84** was obtained as a brown solid.
Yield after purification: 50 % (16 mg).
Melting point > Decomposition at 187 °C.
¹H NMR (400 MHz, acetone-*d*₆) δ 8.40 (s, 1H), 7.63 (bd, *J* = 1.2 Hz, 1H), 6.99 (s, 1H), 6.76 (d, *J* = 3.5 Hz, 1H), 6.56 (dd, *J* = 3.4, 1.8 Hz, 1H).
¹³C NMR (101 MHz, acetone-*d*₆) δ 172.8 (CO), 166.4 (C), 150.4 (C), 144.8 (CH), 139.1 (C), 129.7 (C), 129.0 (CH), 119.0 (C), 117.6 (CH), 112.6 (CH), 110.7 (CH).
HRMS (TOF, ES⁻): Calculated for C₁₁H₆ NO₆ (M-H)⁻: m/z 248.0195. 248.0206 found (deviation 4.4 ppm).

## Claims

1. Derivatives of salicylic acid for the treatment of diseases mediated by GO and/or PRODH2 enzyme activity.

2. Derivatives of salicylic acid according to the preceding claim for the treatment of diseases or conditions linked to an excess of oxalate.

3. Derivatives of salicylic acid according to the preceding claim for the treatment of a disease selected from the group consisting of primary hyperoxaluria (PH-1), secondary hyperoxaluria, idiopathic calcium oxalate urolithiasis.

4. Derivatives of salicylic acid according to the preceding claim for the treatment of primary hyperoxaluria.

5. Derivatives of salicylic acid according to any of the preceding claims with general structure I.

6. Derivatives of salicylic acid according to the preceding claim with general structure A, B or C.

7. Derivatives of salicylic acid according to the preceding claim with general formula 74 or 78.

8. Derivatives of salicylic acid according to claim 6 with general formula MDMG-907, MDMG-911 or MDMG-915.

9. Derivatives of salicylic acid according to any of claims 1 to 3 with general structure II.

10. Derivatives of salicylic acid according to the preceding claim with general structure D, E or F.

11. Derivatives of salicylic acid according to the preceding claim with general formula 73 or 77.

12. Pharmaceutical composition comprising one or more derivatives of salicylic acid according to any of claims 1 to 11 for the treatment of diseases mediated by GO and/or PRODH2 enzyme activity.

13. Combined preparation comprising derivatives of salicylic acid according to any of claims 1 to 11 or a pharmaceutical composition according to the preceding claim, together with other compounds or drugs used for the treatment of diseases mediated by GO and/or PRODH2 enzyme activity.

14. Kit for the preparation of a composition according to claim 12 or of the combined preparation according to claim 13.

15. Method for treating diseases or conditions linked to an excess of oxalate comprising the use of derivatives of salicylic acid according to claims 1 to 10, and/or the composition according to claim 12, and/or the combined preparations according to claim 13, and/or the kit according to claim 14.
